(19) Europäisches Patentamt / European Patent Office / Office européen des brevets

(11) **EP 3 903 855 B1**

(12) **EUROPEAN PATENT SPECIFICATION**

(45) Date of publication and mention of the grant of the patent:
**26.10.2022 Bulletin 2022/43**

(21) Application number: **19904966.9**

(22) Date of filing: **18.12.2019**

(51) International Patent Classification (IPC):
**A61M 1/36** (2006.01)

(52) Cooperative Patent Classification (CPC):
**A61M 1/16; A61M 1/3646; A61M 1/3666; A61M 1/3679; A61M 1/3687; B01J 20/0277; B01J 20/043; B01J 20/06; B01J 20/08; B01J 20/261; B01J 20/262; B01J 20/28069; B01J 20/28078; B01J 20/3007; B01J 20/3021;**

(Cont.)

(86) International application number:
**PCT/JP2019/049671**

(87) International publication number:
**WO 2020/137756 (02.07.2020 Gazette 2020/27)**

(54) **BLOOD PURIFICATION DEVICE AND EXTRACORPOREAL BLOOD CIRCULATION SYSTEM PROVIDED WITH BLOOD COMPONENT ADJUSTER**

BLUTREINIGUNGSVORRICHTUNG UND EXTRAKORPORALES BLUTKREISLAUFSYSTEM MIT BLUTBESTANDTEILREGLER

DISPOSITIF DE PURIFICATION DU SANG ET SYSTÈME DE CIRCULATION SANGUINE EXTRACORPORELLE POURVU D'UN DISPOSITIF DE RÉGLAGE DE COMPOSANT SANGUIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.12.2018 JP 2018241839**

(43) Date of publication of application:
**03.11.2021 Bulletin 2021/44**

(73) Proprietor: **Asahi Kasei Medical Co., Ltd.**
**Tokyo 100-0006 (JP)**

(72) Inventors:
• **OISHI, Teruhiko**
  **Tokyo 100-0006 (JP)**
• **MORITA, Naoki**
  **Tokyo 100-0006 (JP)**
• **OZEKI, Makoto**
  **Tokyo 100-0006 (JP)**
• **KOIZUMI, Toshinori**
  **Tokyo 100-0006 (JP)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) References cited:
WO-A1-2016/103216    WO-A1-2017/082423
WO-A1-2018/180043    CN-A- 105 107 041
DE-A1-102017 216 689    JP-A- 2002 102 335
US-A1- 2016 339 161    US-A1- 2018 326 136

(52) Cooperative Patent Classification (CPC): (Cont.)
A61M 2205/3331; A61M 2205/50; A61M 2205/707;
B01J 2220/46

**Description**

FIELD

The present invention relates to an extracorporeal blood circulation system provided with a blood purification device and a blood component adjuster. More specifically, the invention relates to an extracorporeal blood circulation system which can be safely used, being provided with a blood purification device and a blood component adjuster differing from the blood purification device, wherein dialysis mode is switched to reinfusion mode, and the blood circuit is bypassed, based on pressure loss of the blood purification device and blood component adjuster.

BACKGROUND

Hemodialysis machines are widely used for dialysis of chronic renal failure patients. Dialysis treatment is necessary for life maintenance by removal of ingested water (water removal), especially for patients who do not excrete urine, and therefore the existence of dialysis machines is extremely important.

High performance blood purification devices (dialyzers) have been developed in recent years allowing high-throughput water removal, but for chronic renal failure patients that have impaired renal function there is a need for more functional dialysis treatment in addition to water removal.

Chronic renal failure patients are unable to properly excrete excess phosphorus out of the body, and this leads to gradual internal buildup of phosphorus, causing the condition of hyperphosphatemia. Persistent hyperphosphatemia leads to secondary hyperparathyroidism, resulting in renal osteopathy that is characterized by symptoms such as bone pain, brittleness and deformity, and also proneness to fracture. When accompanied by hypercalcemia, it also increases the risk of cardiac failure due to cardiovascular calcification.

Cardiovascular calcification is one of the most serious complications, and proper control of phosphorus levels in the body is extremely important to prevent hyperphosphatemia in chronic renal failure patients.

PTL 1 describes a blood purification method that includes a blood purification step in which blood is treated using a blood purification device, and a phosphorus adsorption step before and/or after the blood purification step.

In addition, chronic renal failure patients often also suffer cardiac failure due to increased blood volume. Treatments indicated for cardiac failure include DFPP (Double Filtration Plasma Pheresis) and PP (Plasma Perfusion). The psychological and physical load on chronic renal failure patients could be alleviated by the safe use of a device allowing blood components to be adjusted simultaneously with dialysis.

Further relevant prior art is for instance disclosed in documents US2018/326136 Al, DE102017216689 Al, WO 2018/180043 Al, US 2016/339161 Al and CN105107041 A.

It is therefore necessary to provide an extracorporeal blood circulation system that can be safely used and allows adjustment of blood components simultaneously with dialysis.

[CITATION LIST]

[PATENT LITERATURE]

[PTL 1] International Patent Publication No. 2017/082423

SUMMARY

[TECHNICAL PROBLEM]

In light of the prior art described above, the problem to be solved by the invention is to provide an extracorporeal blood circulation system that can be safely used, and that comprises a blood purification device and a blood component adjuster that is separate from the blood purification device.

[SOLUTION TO PROBLEM]

As a result of ardent research with the aim of solving the problem described above, the present inventors have completed this invention after unexpectedly finding that an extracorporeal blood circulation system provided with a blood purification device and a blood component adjuster that is separate from the blood purification device, can be safely used by switching the dialysis mode to reinfusion mode and bypassing the blood circuit, based on pressure loss of the blood purification device and blood component adjuster.

A first extracorporeal blood circulation system according to the present invention comprises the technical features as defined in independent claim 1. Another extracorporeal blood circulation system according to the present invention comprises the technical features as defined in independent claim 2.

[ADVANTAGEOUS EFFECTS OF INVENTION]

The extracorporeal blood circulation system of the invention can be safely used since it switches dialysis mode to reinfusion mode and bypasses the blood circuit based on pressure loss of the blood purification device and blood component adjuster, thereby making it possible to avoid damage to the blood purification device, blood component adjuster and blood circuit (tubing system).

BRIEF DESCRIPTION OF DRAWINGS

Fig. 1 is a schematic diagram of the extracorporeal blood circulation system of Example 1.
Fig. 2 is a schematic diagram of the extracorporeal blood circulation system of Example 2.
Fig. 3 is an overview diagram of a column flow test apparatus in a blood purification device according to an embodiment, with low-phosphorus serum using bovine plasma.

DESCRIPTION OF EMBODIMENTS

The invention will now be explained using embodiments thereof.
The first embodiment of the invention is an extracorporeal blood circulation system running from a blood collection unit (1a) to a blood returning unit (1b), wherein the extracorporeal blood circulation system comprises the following:

a blood component adjuster (4);
a blood purification device (3);
a tubing system (1) comprising a pump (2) for supply of blood from the blood collection unit (1a) to the blood component adjuster (4) in dialysis mode, a valve (8) for supply of physiological saline or air from a tubing system (11) in place of blood, in reinfusion mode, and a pressure gauge (5) for detection of pressure loss of the blood component adjuster (4);
a bypass tubing system (6) comprising a valve (7) for supply of blood to the blood purification device (3) bypassing the blood component adjuster (4), and supply of physiological saline or air in reinfusion mode;
a tubing system (9) which comprises pressure gauges (5', 5") for detecting pressure loss of the blood component adjuster (4) and/or blood purification device (3), and connects the blood component adjuster (4) and the blood purification device (3);
a tubing system (10) comprising a pressure gauge (5‴) for returning blood from the blood purification device (3) to the blood returning unit (1b) and for detecting pressure loss of the blood purification device (3), in dialysis mode, and if necessary a valve (8') for recovering physiological saline or air in the tubing system (11') in place of blood, in reinfusion mode; and
a control unit having a function for switching between the tubing system (1) and the bypass tubing system (6), based on pressure loss of the blood component adjuster (4), and a function for switching between dialysis mode and reinfusion mode, based on pressure loss of the blood purification device (3).

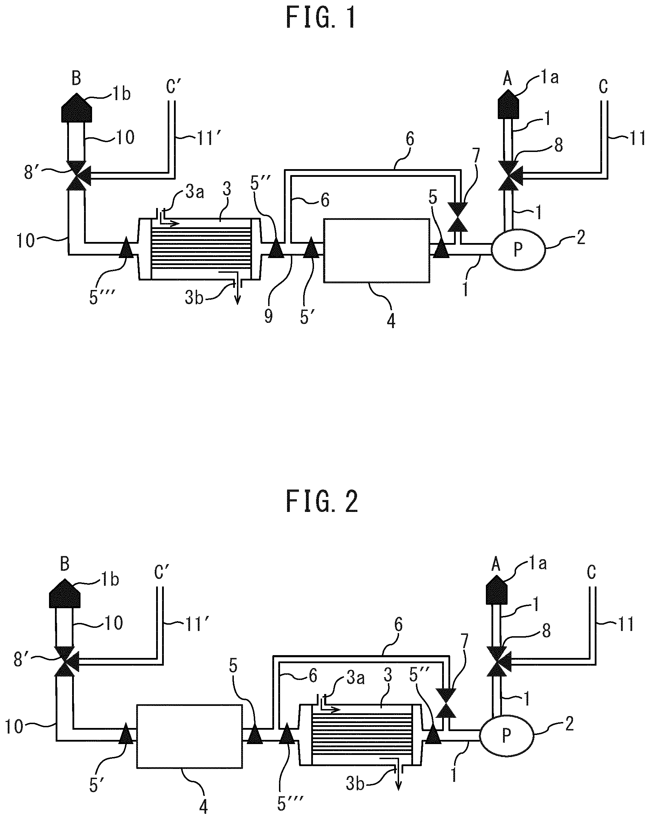

The first embodiment corresponds to Example 1 below, and is shown in overview in Fig. 1.
Another embodiment of the invention is an extracorporeal blood circulation system running from a blood collection unit (1a) to a blood returning unit (1b), wherein the extracorporeal blood circulation system comprises the following:

a blood purification device (3);
a blood component adjuster (4);
a tubing system (1) comprising a pump (2) for supply of blood from the blood collection unit (1a) to the blood purification device (3) in dialysis mode, a valve (8) for supply of physiological saline or air from a tubing system (11) in place of blood, in reinfusion mode, and a pressure gauge (5") for detection of pressure loss of the blood purification device (3);
a bypass tubing system (6) comprising a valve (7) for supply of blood to the blood component adjuster (4) bypassing the blood purification device (3), and supply of physiological saline or air in reinfusion mode;
a tubing system (9) which comprises pressure gauges (5, 5‴) for detecting pressure loss of the blood purification device (3) and/or blood component adjuster (4), and connects the blood purification device (3) and the blood component adjuster (4);
a tubing system (10) comprising a pressure gauge (5') for returning blood from the blood component adjuster (4) to the blood returning unit (1b) and for detecting pressure loss of the blood component adjuster (4), in dialysis mode, and if necessary a valve (8') for recovering physiological saline or air in the tubing system (11') in place of blood, in reinfusion mode; and
a control unit having a function for switching between the tubing system (1) and the bypass tubing system (6), based on pressure loss of the blood purification device (3), and a function for switching between dialysis mode and reinfusion mode, based on pressure loss of the blood component adjuster (4).

The other embodiment corresponds to Example 2 below, and is shown in overview in Fig. 2.

Throughout the present specification, "tubing system" means "blood circuit".

The other embodiment described above has the blood purification device and blood component adjuster of the first embodiment switched.

A method of use and operation of the first embodiment will now be explained with reference to Fig. 1.

The blood collection unit (1a) and blood returning unit (1b) are each inserted into blood vessels (A) and (B) of the patient. In dialysis mode, the blood pressure at the inlet and the filtrate pressure at the outlet of the blood component adjuster (4) are measured by pressure sensors (5, 5') at both ends of the blood component adjuster (4). When the pressure at the inlet of the blood component adjuster (4) has increased to reach a predetermined pressure due to clogging of the blood component adjusting body housed in the blood component adjuster (4), or when the pressure loss has exceeded a predetermined value, a command is given from the control unit (not shown) to open the valve (7) of the bypass tubing system (6), bypassing the blood component adjuster (4). The valve (7) may be connected to any part of the bypass tubing system (6). In dialysis mode, the blood pressure at the inlet and the filtrate pressure at the outlet of the blood purification device (3) are measured by pressure gauges (sensors) (5", 5''') at both ends of the blood purification device (3). When the pressure of the blood purification device (3) has increased to reach a predetermined pressure due to clogging of the hollow fibers, or the pressure loss has exceeded a predetermined value, a command is given from the control unit (not shown) to switch the three-way valve (8) of the tubing system (1) to the tubing system (11), and to switch the three-way valve (8') of the tubing system (10) to the tubing system (11') if it is present, thus changing from dialysis mode (dialysis treatment) to reinfusion mode (returning blood to the patient), or another mode such as stop mode. The tubing system (11) is connected to a reservoir (C) that supplies physiological saline or air, and when present the tubing system (11') is connected to a reservoir (C') that temporarily stores blood and/or physiological saline that has been collected from the blood component adjuster (4), blood purification device (3) and blood circuit, before being returned to the body.

The extracorporeal blood circulation system of this embodiment which comprises the blood purification device blood component adjuster, tubing system (blood circuit) and control unit may be constructed as part of an anticoagulant syringe, an arterial pressure monitor, a venous pressure monitor, a dialysate pressure monitor, a bubble detector, a dialysate supply unit, or a dialysis monitoring device with an alarm function. The dialysis monitoring device allows devices such as electronic parts or pumps to be automatically operated. When multiple combined devices (including a DFPP circuit, for example) are used as the blood component adjuster, devices such as electronic parts and pumps may also be included in the blood component adjuster. Such electronic parts and devices may also be connected to the dialysis monitoring device and automatically controlled. A dialysis monitoring device modified to adapt to the functions of the blood component adjuster may also be used. Alternatively, the functioning and operation of the blood component adjuster may be monitored and controlled by a different apparatus other than a dialysis monitoring device. For safer use, the extracorporeal blood circulation system of this embodiment preferably comprises an electric power generator or battery to allow operation during periods of power outage such as in the event of disaster.

As mentioned above, the other embodiment has the blood purification device and blood component adjuster of the first embodiment switched, and therefore the explanation regarding the method of use and operation of the first embodiment also applies for the method of use and operation of the other embodiment, with reference to Fig. 2.

The modes of operation of the extracorporeal blood circulation system for this embodiment will now be explained.

[Washing or priming mode]

This is a mode in which fine dust, filler solution and air in the blood purification device, blood component adjuster and blood circuit including bypass routes are cleaned and removed with physiological saline, to allow dialysis mode to be started. In the washing or priming mode, the blood collection unit (1a) and blood returning unit (1b) of the blood circuit are connected to reservoirs (C, C') and a waste reservoir, without being inserted into blood vessels (A) and (B) of the patient.

[Dialysis mode]

This is a mode in which the blood collection unit (1a) and blood returning unit (1b) of the blood circuit are inserted into blood vessels (A) and (B) of the patient for dialysis treatment of the patient.

[Reinfusion mode]

This is a mode in which blood in the blood circuit that includes the blood purification device, blood component adjuster and bypass route is returned to the body in a clean and safe manner. Reinfusion methods for reinfusion mode are generally divided into physiological saline replacement methods and air replacement methods. While either one may be used, a physiological saline replacement method is preferred from the viewpoint of safety. Automatic stop mode is engaged upon completion of reinfusion.

[Stop mode]

Stop mode is engaged when a problem has been detected by the dialysis monitoring device during operation of the extracorporeal blood circulation system. Automatic stop mode is engaged when power outage occurs as well. When power is restored after power outage, the mode may be automatically transferred to reinfusion mode, or dialysis mode may be re-engaged. Automatic stop mode is also engaged upon power outage even in reinfusion mode, but reinfusion mode is re-engaged upon power restoration after power outage. Stop mode is maintained when a problem has been detected by the dialysis monitoring device upon restoration of power after power outage.
The blood purification device (3), pressure gauges (sensors) (5, 5', 5", 5‴), bypass tubing system (6), valves (7, 8, 8') and blood component adjuster (4) will now be explained in order.

[Blood purification device (3)]

The blood purification device (3) is not particularly restricted and may be a blood purification module housing a hollow fiber membrane commonly used for hemodialysis treatment, examples of which include blood purification modules used in hemodialysis (HD), ultrafiltration (extracorporeal ultrafiltration, ECUM), hemodialysis filtration (HDF), continuous hemodialysis filtration (CHDF), continuous hemofiltration (CHF) or continuous hemodialysis (CHD). As shown in Fig. 1, dialysate usually flows from the inlet (3a) to the outlet (3b).

[Pressure gauges (sensors)]

The pressure gauges (sensors) (5, 5', 5", 5‴) installed in the tubing systems (1, 9, 10) are not particularly restricted and may be any ones that convert the pressure at the inlets and/or outlets of the blood purification device (3) and blood component adjuster (4) to electrical signals, examples of which include gauge types (strain gauge, metal gauge, semiconductor gauge or semiconductor diaphragm types), electrostatic capacitance types, optical fiber types, oscillating types and pneumatic types. The pressure sensors do not necessarily need to be at both ends of the blood purification device (3) and blood component adjuster (4), and may be present only at one end. The pressure sensors may also be internally embedded in the blood purification device and blood component adjuster.

[Bypass tubing system (6)]

A bypass is provided connecting both ends of the blood component adjuster (4) in Fig. 1 or connecting both ends of the blood purification device (3) in Fig. 2, so that when the inlet pressure of the blood component adjuster (4) or blood purification device (3) increases to reach a predetermined pressure, or when the pressure loss has exceeded a predetermined value, the valve (7) connected to the bypass tubing system (6) is opened allowing blood to flow into the bypass tubing system (6). Since this also allows unexpected pressure increase to be handled, the dialysis treatment can be safely carried out. The material of the bypass tubing system (6) may be the same material as the other tubing systems (1, 10) (blood circuits), or it may be a different material.

[Valves (7, 8, 8')]

The valve (7) connected to the bypass tubing system (6) may be situated at any part of the tubing system, and the tubing system (1) and bypass tubing system (6) may also be switched with a three-way valve. The valve (7) is a device functioning to open and close liquid flow to the bypass route, and it is electronically controllable. The flow volume to the bypass route can be adjusted by the semi-open state of the valve. Adjustment from the semi-open state to the closed state, and from the closed state to the semi-open state, is also possible.

The valve (8) in the tubing system (1), and if necessary the valve (8') in the tubing system (10), are devices for switching between dialysis mode and reinfusion mode, and for supplying or collecting physiological saline or air in reinfusion mode, and they are also electronically controllable. The valves may also be three-way valves.

[Blood component adjuster (4)]

The blood component adjuster (4) is separate from the blood purification device (3). The blood component adjuster (4) is a device allowing removal or supply of biological blood components, and it is not particularly restricted. It may be either a single device (such as a hydrogen feeder or cytokine remover), or a combination of multiple devices (such as a DFPP). Blood components include water, blood plasma, blood cells (erythrocytes, leukocytes, lymphocytes, platelets, etc.), proteins (albumin, fibrinogen, immunoglobulins, etc.), saccharides (glucose, glycogen, etc.), lipids (triglycerides, phospholipids, cholesterol, etc.), inorganic salts (salts comprising chlorine, bicarbonic acid, sulfuric acid, phosphoric acid, calcium, sodium, potassium, magnesium, iron, copper and phosphorus, for example), amino acids, hormones, vitamins, insulin, hydrogen, nitrogen, oxygen, carbon dioxide, urea, creatine, creatinine, ammonia, antibodies, pathogens, bacteria, viruses, parasites, tumor cells, cytokines (including proteins with molecular weights of 8 to 30 kDa that participate in cell proliferation, differentiation and functional expression, such as interleukin-1$\beta$, interleukin-6, interleukin-8 and TNF$\alpha$), exosomes, microparticles, RNA and MicroRNA. The blood component adjuster removes pathogenic (or related) substances, or supplies substances for treatment of disease.

[Blood component adjusting body]

The blood component adjuster may contain a blood component adjusting body. The blood component adjusting body may be a porous molded body having the function of removing or supplying a blood component, examples of which include active carbon, membranes (such as hollow fiber membranes, flat (spiral or pleated) membranes, tubular membranes and monolithic ceramic films), beads and fibers. When the blood component adjuster (4) is a hydrogen feeder, for example, a gas exchange membrane (artificial lung) may be used as the blood component adjusting body. For removal of the target substance it is sufficient to add a suitable ligand to the porous molded body. A ligand having an electrostatic bonding function (such as polyacrylic acid), a ligand having hydrophobic bonds (such as a hexadecyl group or petroleum pitch-based active carbon) or a ligand having a complex bond (such as polymyxin B) may be used. For example, if polyacrylic acid is used as the ligand it is possible to remove cholesterol, or if polymyxin B is used as the ligand it is possible to remove endotoxins. If a polyester is used as a fiber material or cellulose diacetate is used as a bead material, it is possible to remove leukocytes.

[Porous molded body]

The porous molded body of this embodiment is composed of a porous molded body-forming polymer and a hydrophilic polymer, or a porous molded body-forming polymer, a hydrophilic polymer and an inorganic ion adsorbent. If the porous molded body includes an inorganic ion adsorbent, then the total volume of pores with pore diameters of 1 nm to 80 nm, as measured by the nitrogen gas adsorption method, is 0.05 cm$^3$/g to 0.7 cm$^3$/g, preferably 0.1 cm$^3$/g to 0.6 cm$^3$/g and more preferably 0.2 cm$^3$/g to 0.5 cm$^3$/g, per unit mass of the inorganic ion adsorbent.

The pore volume is obtained by measuring the freeze-dried porous molded body by the nitrogen gas adsorption method and calculating by the BJH method.

The sum Va of the pore volumes per unit mass of the inorganic ion adsorbent is determined by the following formula (1):

$$Va = Vb/Sa \times 100 \quad (1)$$

where Vb (cm$^3$/g) is the pore volume per unit mass of the porous molded body calculated for the dried porous molded body and Sa (mass%) is the loading mass of the inorganic ion adsorbent in the porous molded body.

The loading mass (mass%) Sa of the inorganic ion adsorbent in the porous molded body is determined by the following formula (2):

$$Sa = Wb/Wa \times 100 \quad (2)$$

where Wa (g) is the mass of the porous molded body when dry and Wb (g) is the ash content mass.

The ash content is the portion remaining after the porous molded body has been fired at 800°C for 2 hours.

Since the pore volume of the porous molded body measured by the nitrogen gas adsorption method is a value primarily reflecting the pore volume of the inorganic ion adsorbent in the porous molded body, a larger value represents higher diffusion efficiency of ions into the inorganic ion adsorbent, and higher adsorption capacity.

If the sum of the pore volumes per unit mass of the inorganic ion adsorbent is smaller than 0.05 cm$^3$/g, the pore volume of the inorganic ion adsorbent will be reduced and the adsorption capacity will be significantly lower. If the value is higher than 0.7 cm$^3$/g, on the other hand, the bulk density of the inorganic ion adsorbent will increase and the viscosity of the stock solution slurry will increase, thereby hampering granulation.

The area-to-weight ratio of the porous molded body measured by the nitrogen gas adsorption method is preferably 50 m$^2$/g to 400 m$^2$/g, more preferably 70 m$^2$/g to 350 m$^2$/g and even more preferably 100 m$^2$/g to 300 m$^2$/g.

The area-to-weight ratio is obtained by measuring the freeze-dried porous molded body by the nitrogen gas adsorption method and calculating by the BET method.

Since the area-to-weight ratio of the porous molded body measured by the nitrogen gas adsorption method is a value primarily reflecting the area-to-weight ratio of the inorganic ion adsorbent in the porous molded body, a larger value represents a greater number of ion adsorption sites and higher adsorption capacity.

If the area-to-weight ratio of the porous molded body is smaller than 50 m$^2$/g, the number of adsorption sites of the inorganic ion adsorbent will be lower and the adsorption capacity will be significantly reduced. If the value is higher than 400 m$^2$/g, on the other hand, the bulk density of the inorganic ion adsorbent will increase and the viscosity of the stock solution slurry will increase, thereby hampering granulation.

The loading mass of the inorganic ion adsorbent in the porous molded body is preferably 30 mass% to 95 mass%, more preferably 40 mass% to 90 mass% and even more preferably 50 mass% to 80 mass%.

If the loading mass is less than 30 mass%, the contact frequency between the ions to be adsorbed and the inorganic ion adsorbent as the adsorption substrate will tend to be insufficient, while if it is greater than 95 mass%, the strength of the porous molded body will tend to be lacking.

The porous molded body preferably has a mean particle size of 100 μm to 2500 μm and is essentially in the form of spherical particles, the mean particle size being preferably 150 μm to 2000 μm, more preferably 200 μm to 1500 μm and even more preferably 300 μm to 1000 μm.

The porous molded body is preferably in the form of spherical particles, although the spherical particles are not limited to being only true spherical and may also be elliptical spherical.

The mean particle size is the median diameter of the sphere-equivalent size determined from the angular distribution of the intensity of scattered light due to laser light diffraction, assuming the porous molded body to be spherical.

If the mean particle size is 100 μm or greater, pressure loss will be low when the porous molded body is packed into a container such as a column or tank, making it suitable for high-speed water treatment. If the mean particle size is 2500 μm or smaller, on the other hand, the surface area of the porous molded body can be increased when it has been packed into a column or tank, allowing reliable adsorption of ions even with high-speed liquid flow treatment.

[Inorganic ion adsorbent]

The inorganic ion adsorbent composing the porous molded body is an inorganic substance that exhibits an ion adsorption phenomenon or ion-exchange phenomenon.

Examples of natural inorganic ion adsorbents include mineral substances such as zeolite and montmorillonite.

Specific examples of mineral substances include kaolin minerals having a single layer lattice with aluminosilicates, muscovite, glauconite, kanuma soil, pyrophyllite and talc having a 2-layer lattice structure, and feldspar, zeolite and montmorillonite having a three-dimensional frame structure.

Examples of synthetic-based inorganic ion adsorbents include metal oxides, polyvalent metal salts and insoluble hydrous oxides. Metal oxides include complex metal oxides, composite metal hydroxides and metal hydrous oxides.

From the viewpoint of adsorption performance for the target of absorption, and phosphorus, the inorganic ion adsorbent preferably contains at least one metal oxide represented by the following formula (I):

$$MN_xO_n \cdot mH_2O \qquad (I)$$

{where x is 0 to 3, n is 1 to 4, m is 0 to 6, and M and N are metal elements selected from the group consisting of Ti, Zr, Sn, Sc, Y, La, Ce, Pr, Nd, Sm, Eu, Gd, Tb, Dy, Ho, Er, Tm, Yb, Lu, Al, Si, Cr, Co, Ga, Fe, Mn, Ni, V, Ge, Nb and Ta, and are different from each other}.

The metal oxide may be a non-water-containing (non-hydrated) metal oxide where m in formula (I) is 0, or it may be a hydrous metal oxide (hydrated metal oxide) wherein m is a numerical value other than 0.

A metal oxide where x in formula (I) is a numerical value other than 0 is a complex metal oxide represented by the aforementioned chemical formula in which each metal element is evenly distributed in a regular manner throughout all of the oxides, and the compositional ratio of the metal elements in the metal oxide is constant.

Specific ones include nickel ferrite ($NiFe_2O_4$) or hydrous ferrite of zirconium ($Zr \cdot Fe_2O_4 \cdot mH_2O$, where m is 0.5 to 6), which form a perovskite structure or spinel structure.

The inorganic ion adsorbent may also contain more than one type of metal oxide represented by formula (I).

From the viewpoint of excellent adsorption performance for components to be adsorbed, and especially phosphorus, a metal oxide as the inorganic ion adsorbent is preferably selected from among the following groups (a) to (c):

(a) hydrated titanium oxide, hydrated zirconium oxide, hydrated tin oxide, hydrated cerium oxide, hydrated lanthanum oxide and hydrated yttrium oxide,
(b) complex metal oxides comprising at least one metal element selected from the group consisting of titanium, zirconium, tin, cerium, lanthanum and yttrium and at least one metal element selected from the group consisting of aluminum, silicon and iron, and
(c) activated alumina.

It may be a material selected from among any of groups (a) to (c), or materials selected from among any of groups (a) to (c) may be used in combination, or materials of each of groups (a) to (c) may be used in combination. When materials are used in combination, they may be a mixture of two or more materials selected from among any of groups (a) to (c), or they may be a mixture of two or more materials selected from among two or more of groups (a) to (c).

From the viewpoint of low cost and high adsorption properties, the inorganic ion adsorbent may contain aluminum sulfate-added activated alumina.

From the viewpoint of inorganic ion adsorption properties and production cost, the inorganic ion adsorbent is more preferably one having a metal element other than M and N in solid solution in addition to the metal oxide represented by formula (I).

For example, it may be one with iron in solid solution with hydrated zirconium oxide represented by $ZrO_2 \cdot mH_2O$ (where m is a numerical value other than 0).

Examples of salts of polyvalent metals include hydrotalcite-based compounds represented by the following formula (II):

$$M^{2+}_{(1-p)}M^{3+}_{p}(OH^-)(2+p-q)(A^{n-})_{q/r} \qquad \text{(II)}$$

{where $M^{2+}$ is at least one divalent metal ion selected from the group consisting of $Mg^{2+}$, $Ni^{2+}$, $Zn^{2+}$, $Fe^{2+}$, $Ca^{2+}$ and $Cu^{2+}$, $M^{3+}$ is at least one trivalent metal ion selected from the group consisting of $Al^{3+}$ and $Fe^{3+}$, $A^{n-}$ is an n-valent anion, $0.1 \le p \le 0.5$, $0.1 \le q \le 0.5$, and r is 1 or 2}.

A hydrotalcite-based compound represented by formula (II) is preferred because it is inexpensive as an inorganic ion adsorbent and has high adsorption properties.

Examples of insoluble hydrous oxides include insoluble heteropolyacid salts and insoluble hexacyanoferrates.

A metal carbonate as the inorganic ion adsorbent has excellent performance from the viewpoint of adsorption, but using a carbonate requires consideration from the viewpoint of elution.

From the viewpoint of allowing ion-exchange reaction with the carbonate ion, the metal carbonate may include at least one type of metal carbonate represented by the following formula (III):

$$QyRz(CO_3)s \cdot tH_2O \qquad \text{(III)}$$

{where y is 1 or 2, Z is 0 or 1, s is 1 to 3, t is 0 to 8, and Q and R are metal elements selected from the group consisting of Mg, Ca, Sr, Ba, Sc, Mn, Fe, Co, Ni, Ag, Zn, Y, La, Ce, Pr, Nd, Sm, Eu, Gd, Tb, Dy, Ho, Er, Tm, Yb and Lu, and are different from each other}.

The metal carbonate may be a non-hydrous (non-hydrated) metal carbonate where t in formula (III) is 0, or it may be a hydrate where t is an integer other than 0.

From the viewpoint of low elution and excellent adsorption properties for phosphorus, boron, fluorine and/or arsenic, the inorganic ion adsorbent is preferably selected from among the following group (d):

(d) magnesium carbonate, calcium carbonate, strontium carbonate, barium carbonate, scandium carbonate, manganese carbonate, iron carbonate, cobalt carbonate, nickel carbonate, silver carbonate, zinc carbonate, yttrium carbonate, lanthanum carbonate, cerium carbonate, praseodymium carbonate, neodymium carbonate, samarium carbonate, europium carbonate, gadolinium carbonate, terbium carbonate, dysprosium carbonate, holmium carbonate, erbium carbonate, thulium carbonate, ytterbium carbonate and lutetium carbonate.

The inorganic ion adsorption mechanism for the metal carbonate is expected to include elution of the metal carbonate and recrystallization of inorganic ions and metal ions on the metal carbonate, and therefore a higher degree of solubility of the metal carbonate is anticipated to result in higher inorganic ion adsorption and more excellent adsorption performance. Metal elution from the inorganic ion adsorbent is also a concern, and therefore careful study is necessary for uses where metal elution may be problem.

The inorganic ion adsorbent composing the porous molded body may also contain contaminating impurity elements that are present due to the production process, in ranges that do not interfere with functioning of the porous molded body. Examples of potentially contaminating impurity elements include nitrogen (in the form of nitric acid, nitrous acid or ammonium), sodium, magnesium, sulfur, chlorine, potassium, calcium, copper, zinc, bromine, barium and hafnium.

The inorganic ion adsorbent composing the porous molded body may also contain contaminating impurity elements that are present due to the production process, in ranges that do not interfere with functioning of the porous molded body. Examples of potentially contaminating impurity elements include nitrogen (in the form of nitric acid, nitrous acid or ammonium), sodium, magnesium, sulfur, chlorine, potassium, calcium, copper, zinc, bromine, barium and hafnium.

The method of replacement to organic liquid is not particularly restricted, and it may be centrifugal separation and filtration after dispersing the water-containing inorganic ion adsorbent in an organic liquid, or passage of an organic liquid after filtration with a filter press. For a higher replacement rate, it is preferred to repeat a method of filtration after dispersion of the inorganic ion adsorbent in an organic liquid.

The replacement rate of water to organic liquid during production may be 50 mass% to 100 mass%, preferably 70 mass% to 100 mass% and more preferably 80 mass% to 100 mass%.

The organic liquid replacement rate is the value represented by the following formula (3):

$$Sb = 100 - Wc \ (3)$$

where Sb (mass%) is the replacement rate to organic liquid and Wc (mass%) is the moisture content of the filtrate after treating the water-containing inorganic ion adsorbent with the organic liquid.

The moisture content of the filtrate after treatment with the organic liquid can be determined by measurement by the Karl Fischer method.

Drying after replacement of the water in the inorganic ion adsorbent with organic liquid can inhibit aggregation during drying, can increase the pore volume of the inorganic ion adsorbent and can increase the adsorption capacity.

If the replacement rate of the organic liquid is less than 50 mass%, the aggregation suppressing effect during drying will be reduced and the pore volume of the inorganic ion adsorbent will not increase.

[Porous molded body-forming polymer]

The porous molded body-forming polymer of this embodiment may be any polymer capable of forming a porous molded body, examples of which include various types such as polysulfone-based polymers, polyvinylidene fluoride-based polymers, polyvinylidene chloride-based polymers, acrylonitrile-based polymers, polymethyl methacrylate-based polymers, polyamide-based polymers, polyimide-based polymers, cellulosic polymers, ethylene-vinyl alcohol copolymer-based polymers, polyaryl ether sulfones, polypropylene-based polymers, polystyrene-based polymers and polycarbonate-based polymers. Among these, aromatic polysulfones are preferred for excellent thermostability, acid resistance, alkali resistance and mechanical strength.

Aromatic polysulfones to be used for the embodiment include those having repeating units represented by the following formula (IV):

$$-O-Ar-C(CH_3)_2-Ar-O-Ar-SO_2-Ar- \qquad (IV)$$

{where Ar is a disubstituted phenyl group at the para position}
or the following formula (V):

$$-O-Ar-SO_2-Ar- \qquad (V)$$

{where Ar is a disubstituted phenyl group at the para position}. The polymerization degree and molecular weight of the aromatic polysulfone are not particularly restricted.

[Hydrophilic polymer]

A hydrophilic polymer used to form the porous molded body of the embodiment is not particularly restricted so long as it is a biocompatible polymer that swells but does not dissolve in water, and examples include polymers having one or more sulfonic acid, carboxyl, carbonyl, ester, amino, amide, cyano, hydroxyl, methoxy, phosphate, oxyethylene, imino, imide, iminoether, pyridine, pyrrolidone, imidazole or quaternary ammonium groups.

When the porous molded body-forming polymer is an aromatic polysulfone, a polyvinylpyrrolidone (hereunder also referred to as "PVP")-based polymer is most preferred as the hydrophilic polymer.

Polyvinylpyrrolidone-based polymers include vinylpyrrolidone-vinyl acetate copolymer, vinylpyrrolidone-vinylcaprolactam copolymer and vinylpyrrolidone-vinyl alcohol copolymer, and preferably at least one of these is used. From the viewpoint of compatibility with the polysulfone-based polymer, the most suitable ones for use are polyvinylpyrrolidone, vinylpyrrolidone-vinyl acetate copolymer and vinylpyrrolidone-vinylcaprolactam copolymer.

The porous molded body is preferably coated with a biocompatible polymer, the biocompatible polymer preferably being selected from the group consisting of polymethoxyethyl acrylate (PMEA) and polyvinylpyrrolidone (PVP)-based polymers.

[Polymethoxyethyl acrylate (PMEA)]

The biocompatibility of PMEA is described in detail in "Artificial organ surface-biocompatibilizing materials", Tanaka, K., BIO INDUSTRY, Vol. 20, No. 12, 59-70 2003.

This article describes preparing PMEA, and an acrylate-based polymer with a different side chain structure for comparison, and evaluating platelets, leukocytes, complement and coagulation markers during circulation of blood, and it is stated that "the PMEA surface had minor activation of blood components compared to other polymers, while the PMEA surface had excellent blood compatibility due to a significantly low level of human platelet adhesion and low morphological changes in the adhered platelets".

Presumably, therefore, PMEA has good biocompatibility not simply because it has ester groups in the structure, but in addition the state of water molecules adsorbed onto the surface also affects its biocompatibility in a major way.

It is known that in the ATR-IR method, waves impinging on a sample are reflected after entering into the sample to a small degree, such that infrared absorption in the region of the entering depth can be measured, but the present inventors have found that the region of measurement in the ATR-IR method is essentially equal to the depth of the "surface layer" that corresponds to the surface of the porous molded body. That is, it is believed that the biocompatibility in a region at approximately equal depth as the ATR-IR measurement region governs the biocompatibility of the porous molded body, and that the presence of PMEA in that region can provide a blood purification device with consistent biocompatibility.

If the surface of the porous molded body is coated with PMEA, then generation of microparticles from the blood purification device after long-term storage can also be inhibited.

The measuring region by ATR-IR depends on the wavelength and incident angle of infrared light in air, the refractive index of the prism and the refractive index of the sample, but it will usually be a region of within 1 $\mu$m from the surface.

The presence of PMEA on the surface of the porous molded body can be confirmed by thermal decomposition gas chromatography-mass spectrometry of the porous molded body. The presence of PMEA is estimated using the peak near 1735 cm$^{-1}$ on the infrared absorption curve from total reflection infrared absorption (ATR-IR) measurement of the surface of the porous molded body, although neighboring peaks can arise due to other substances. Thermal decomposition gas chromatography-mass spectrometry allows the presence of PMEA to be known by confirming PMEA-derived 2-methoxyethanol.

PMEA has a characteristic solubility in solvents. For example, PMEA does not dissolve in a 100% ethanol solvent but has a range of solubility in a water/ethanol mixed solvent, depending on the mixing ratio. If the mixing ratio is in the soluble range, the peak intensity of the PMEA-attributed peak (near 1735 cm$^{-1}$) is higher with a larger amount of water.

For a porous molded body comprising PMEA on the surface, the variation in water permeability is minimal and product design is simpler, due to lower variation in pore sizes on the surface. The porous molded body has PMEA on the surface, but when the PMEA has been coated onto the porous molded body it is assumed that the PMEA adheres as an ultra-thin film, coating the porous molded body surface essentially without blocking the pores. PMEA is especially preferred because of its small molecular weight and short molecular chains, which makes it less likely to form a thick coating film structure or to alter the structure of the porous molded body. PMEA is also preferred because it has high compatibility with other substances, allowing it to be evenly coated onto the porous molded body surface and helping to improve the biocompatibility.

The weight-average molecular weight of the PMEA can be measured by gel permeation chromatography (GPC), for example.

The method of including PMEA on the surface of the porous molded body may be a method of coating by flowing a PMEA-dissolved coating solution from the top of a column (vessel) packed with the porous molded body.

[Polyvinylpyrrolidone (PVP)-based polymer]

The polyvinylpyrrolidone (PVP)-based polymer is not particularly restricted, but polyvinylpyrrolidone (PVP) is suitable for use.

[Phosphorus adsorption performance of porous molded body]

The porous molded body can be suitably used for adsorption of phosphorus during hemodialysis of a dialysis patient. The composition of blood is categorized into blood plasma components and blood cell components, with the blood plasma components comprising 91% water, 7% proteins, and lipid components and inorganic salts, and with phosphorus in the blood being present as phosphate ions among the blood plasma components. The blood cell components are composed of 96% erythrocytes, 3% leukocytes and 1% platelets, the sizes of erythrocytes being 7 to 8 $\mu$m in diameter, the sizes of leukocytes being 5 to 20 $\mu$m in diameter and the sizes of platelets being 2 to 3 $\mu$m in diameter.

Since the most common pore size of a porous molded body measured by a mercury porosimeter is 0.08 to 0.70 $\mu$m, and consequently the abundance of the inorganic ion adsorbent on the outer surface is high, this allows phosphorus ions to be reliably adsorbed even by high-speed liquid flow treatment, and also allows excellent penetration, diffusion and adsorption of phosphorus ions into the porous molded body. There is also no reduction in blood flow by clogging with blood cell components.

The surface of the porous molded body has a biocompatible polymer, allowing it to be used as a more suitable phosphorus adsorbent for blood treatment.

If the device comprises a porous molded body with the most common pore size being 0.08 to 0.70 $\mu$m and the surface of the porous molded body has a biocompatible polymer, then phosphorus ions in blood will be selectively and reliably adsorbed, so that the phosphorus concentration in blood returning to the body will be nearly 0. By returning essentially phosphorus-free blood to the body, presumably phosphorus will more actively move into the blood from intracellular or extracellular regions, for a greater refilling effect.

By inducing a refilling effect supplementing phosphorus in the blood, it may even be possible to eliminate phosphorus present in extracellular fluid or in cells, which normally cannot be eliminated.

Thus, phosphorus levels in the blood of a dialysis patient can be properly managed without taking oral phosphorus adsorbents, or by taking only small amounts (auxiliary usage), thus avoiding side-effects in dialysis patients.

[Method for producing porous molded body]

A method for producing a porous molded body will now be described.

The method for producing a porous molded body includes, for example, (1) a step of drying an inorganic ion adsorbent, (2) a step of pulverizing the inorganic ion adsorbent obtained in step (1), (3) a step of mixing the inorganic ion adsorbent obtained in step (2), a good solvent for the porous molded body-forming polymer, a porous molded body-forming polymer and a hydrophilic polymer (water-soluble polymer) to prepare a slurry, (4) a step of molding the slurry obtained in step (3), and (5) a step of coagulating the molded article obtained in step (4) in a poor solvent.

[Step (1): Inorganic ion adsorbent drying step]

In step (1), the inorganic ion adsorbent is dried to obtain a powder. In order to inhibit aggregation during the drying, preferably the drying during production is carried out after replacing the moisture with an organic liquid. The organic liquid is not particularly restricted so long as it has an effect of inhibiting aggregation of the inorganic ion adsorbent, but it is preferred to use a liquid with high hydrophilicity. Examples include alcohols, ketones, esters and ethers.

The replacement rate to the organic liquid may be 50 mass% to 100 mass%, preferably 70 mass% to 100 mass% and more preferably 80 mass% to 100 mass%.

The method of replacement to organic liquid is not particularly restricted, and it may be centrifugal separation and filtration after dispersing the water-containing inorganic ion adsorbent in an organic liquid, or passage of an organic liquid after filtration with a filter press. For a higher replacement rate, it is preferred to repeat a method of filtration after dispersion of the inorganic ion adsorbent in an organic liquid.

The replacement rate to the organic liquid can be determined by measurement of the filtrate moisture content by the Karl Fischer method.

Drying after replacement of the water in the inorganic ion adsorbent with organic liquid can inhibit aggregation during drying, can increase the pore volume of the inorganic ion adsorbent and can increase the adsorption capacity.

If the replacement rate of the organic liquid is less than 50 mass%, the aggregation suppressing effect during drying will be reduced and the pore volume of the inorganic ion adsorbent will not increase.

[Step (2): Inorganic ion adsorbent pulverizing step]

In step (2), the inorganic ion adsorbent powder obtained from step (1) is pulverized. The pulverizing method is not particularly restricted, and may be dry grinding or wet grinding.

A dry grinding method is not particularly restricted, and it may be one employing an impact crusher such as a hammer mill, an airflow pulverizer such as a jet mill, a medium pulverizer such as a ball mill or a compression pulverizer such as a roller mill.

An airflow pulverizer is preferred among these because it can create a sharp particle size distribution of the pulverized inorganic ion adsorbent.

A wet grinding method is not particularly restricted so long as it allows pulverizing and mixing together of the inorganic ion adsorbent and the good solvent for the polymer resin, and it may employ means used in physical pulverizing methods such as pressurized disruption, mechanical grinding or ultrasonic treatment.

Specific examples of pulverizing and mixing means include blenders such as generator shaft homogenizers and Waring blenders, medium agitation mills such as sand mills, ball mills, attritors and bead mills, and jet mills, mortar/pestle combinations, kneaders and sonicators.

A medium agitation mill is preferred for high pulverizing efficiency and to allow pulverizing to a highly viscous state.

The ball diameter used in a medium agitation mill is not particularly restricted but is preferably 0.1 mm to 10 mm. If the ball diameter is 0.1 mm or greater, the ball mass will be sufficient to provide pulverizing force and high pulverizing efficiency, while a ball diameter of 10 mm or smaller will result in excellent fine pulverizing power.

The material of the ball used in a medium agitation mill is not particularly restricted, and it may be a metal such as iron or stainless steel, or a ceramic which is an oxide such as alumina or zirconia or a non-oxide such as silicon nitride or silicon carbide. Zirconia is superior among these for its excellent abrasion resistance, and from the viewpoint of low contamination (wear contamination) into products.

After pulverizing, a filter or the like is preferably used for filtration purification with the inorganic ion adsorbent in a fully dispersed state in the good solvent for the porous molded body-forming polymer.

The particle size of the pulverized and purified inorganic ion adsorbent is 0.001 to 10 $\mu$m, preferably 0.001 to 0.1 $\mu$m and more preferably 0.01 to 0.1 $\mu$m. A smaller particle size is more favorable for uniformly dispersing the inorganic ion adsorbent in the membrane-forming solution. It tends to be difficult to produce uniform microparticles with sizes of smaller than 0.001 $\mu$m. With an inorganic ion adsorbent exceeding 10 $\mu$m, it tends to be difficult to stably produce a porous molded body.

[Step (3): Slurry preparation step]

In step (3), the inorganic ion adsorbent obtained in step (2), a good solvent for the porous molded body-forming polymer, a porous molded body-forming polymer and, depending on the case, a water-soluble polymer are mixed to prepare a slurry.

The good solvent for the porous molded body-forming polymer used in step (2) and step (3) is not particularly restricted so long as it stably dissolves the porous molded body-forming polymer at greater than 1 mass% under the production conditions for the porous molded body, and any conventionally known one may be used.

Examples of good solvents include N-methyl-2-pyrrolidone (NMP), N,N-dimethylacetamide (DMAC) and N,N-dimethylformamide (DMF).

The good solvent used may be a single one alone, or two or more may be used in admixture.

The amount of porous molded body-forming polymer added in step (3) may be such that the proportion of porous molded body-forming polymer/(porous molded body-forming polymer + water-soluble polymer + good solvent for porous molded body-forming polymer) is preferably 3 mass% to 40 mass% and more preferably 4 mass% to 30 mass%. If the porous molded body-forming polymer content is 3 mass% or greater a porous molded body with high strength can be obtained, and if it is 40 mass% or lower, a porous molded body with high porosity can be obtained.

While addition of a water-soluble polymer is not absolutely necessary in step (3), addition can yield a homogeneous porous molded body comprising a filamentous structure that forms a three-dimensional connected network structure on the outer surface and interior of the porous molded body, and a porous molded body can be obtained and reliable ion adsorption even with high-speed liquid flow treatment.

The water-soluble polymer used in step (3) is not particularly restricted so long as it is compatible with the good solvent for the porous molded body-forming polymer, and with the porous molded body-forming polymer.

A natural polymer, semisynthetic polymer or synthetic polymer may be used as the water-soluble polymer.

Examples of natural polymers include guar gum, locust bean gum, carrageenan, gum arabic, tragacanth, pectin, starch, dextrin, gelatin, casein and collagen.

Examples of semisynthetic polymers include methyl cellulose, ethyl cellulose, hydroxyethyl cellulose, ethylhydroxyethyl cellulose, carboxymethyl starch and methyl starch.

Examples of synthetic polymers include polyvinyl alcohol, polyvinylpyrrolidone (PVP), polyvinyl methyl ether, carboxyvinyl polymer, sodium polyacrylate, and polyethylene glycols such as tetraethylene glycol and triethylene glycol.

A synthetic polymer is preferred from the viewpoint of increasing the loading capacity of the inorganic ion adsorbent, while polyvinylpyrrolidone (PVP) or a polyethylene glycol is preferred from the viewpoint of increasing the porosity.

The weight-average molecular weight of the polyvinylpyrrolidone (PVP) or polyethylene glycol is preferably 400 to 35,000,000, more preferably 1,000 to 1,000,000 and even more preferably 2,000 to 100,000.

If the weight-average molecular weight is 400 or greater, a porous molded body with high surface openness will be obtained, and if it is 35,000,000 or lower, the viscosity of the slurry during molding will be low, tending to facilitate the molding.

The weight-average molecular weight of the water-soluble polymer can be measured by dissolving the water-soluble polymer in a predetermined solvent and analyzing it by gel permeation chromatography (GPC).

The amount of water-soluble polymer added may be such that the proportion of water-soluble polymer/(water-soluble polymer + porous molded body-forming polymer + good solvent for porous molded body-forming polymer) is preferably 0.1 mass% to 40 mass%, more preferably 0.1 mass% to 30 mass% and even more preferably 0.1 mass% to 10 mass%.

If the amount of water-soluble polymer added is 0.1 mass% or greater, it will be possible to uniformly obtain a porous molded body that includes a filamentous structure forming a network structure that is three-dimensionally connected on the outer surface and interior of the porous molded body. If the amount of water-soluble polymer added is 40 mass% or lower, the open area ratio on the outer surface will be satisfactory and the abundance of the inorganic ion adsorbent on the outer surface of the porous molded body will be high, to obtain a porous molded body that can reliably adsorb ions even with high-speed liquid flow treatment.

[Step (4): Molding step]

In step (4), the slurry obtained in step (3) (molding slurry) is molded. The molding slurry is a mixed slurry comprising the porous molded body-forming polymer, the good solvent for the porous molded body-forming polymer, the inorganic ion adsorbent and a water-soluble polymer.

The form of the porous molded body of the embodiment may be any desired form such as particulate, filamentous, sheet-like, hollow fiber-like, cylindrical or hollow cylindrical, depending on the method of molding the molding slurry.

There are no particular restrictions on the method of molding a particulate form, such as spherical particles, and for example, it may be a rotation nozzle method in which the molding slurry housed in a vessel is ejected from nozzles provided on the side wall of the rotating vessel to form droplets. The rotating nozzle method allows molding into a particulate form with a uniform particle size distribution.

More specifically, the method may be atomization of the molding slurry from single-fluid or double-fluid nozzles for coagulation in a coagulating bath.

The nozzle diameters are preferably 0.1 mm to 10 mm and more preferably 0.1 mm to 5 mm. The droplets will be more easily ejected if the nozzle diameters are at least 0.1 mm, and the particle size distribution can be made uniform if it is 10 mm or smaller.

The centrifugal force is represented as the centrifugal acceleration, and it is preferably 5 G to 1500 G, more preferably 10 G to 1000 G and even more preferably 10 G to 800 G.

If the centrifugal acceleration is 5 G or greater the formation and ejection of the droplets will be facilitated, and if it is 1500 G or lower the molding slurry will be discharged without becoming filamentous, and widening of the particle size distribution can be inhibited. A narrow particle size distribution will result in uniform water flow channels when the porous molded body is packed into the column, providing an advantage whereby even when ultra high-speed water flow treatment is used there is no leakage of ions (the target of adsorption) from the start of water flow.

A method of molding into a filamentous or sheet form may be a method of extruding the molding slurry from a spinneret or die having that shape, and coagulating it in a poor solvent.

A method of molding into a hollow fiber porous molded body may be molding in the same manner as a method of molding the porous molded body into a filamentous or sheet form, but using a spinneret with an annular orifice.

A method of molding the porous molded body into a cylindrical or hollow cylindrical form, when extruding the molding slurry from a spinneret, may be cutting while coagulating in a poor solvent, or coagulation into a filamentous form followed by cutting.

[Step (5): Coagulation step]

In step (5), the molded article with promoted coagulation obtained in step (4) is further coagulated in a poor solvent to obtain a porous molded body.

<Poor solvent>

The poor solvent for step (5) may be a solvent with a solubility of 1 mass% or lower for the porous molded body-forming polymer under the conditions in step (5), and examples include water, alcohols such as methanol and ethanol, ethers, and aliphatic hydrocarbons such as *n*-hexane and *n*-heptane. Water is most preferred as the poor solvent.
In step (5), the good solvent is carried over from the preceding steps, causing variation in the concentration of the good solvent at the start and end points of the coagulation step. The poor solvent may therefore have the good solvent added beforehand, and preferably the coagulation step is carried out while managing the concentration by separate addition of water or the like so as to maintain the initial concentration.
By adjusting the concentration of the good solvent it is possible to control the structure (the outer surface open area ratio and particle shapes) of the porous molded body.
When the poor solvent is water or a mixture of water with the good solvent for the porous molded body-forming polymer, the content of the good solvent for the porous molded body-forming polymer with respect to the water in the coagulation step is preferably 0 to 80 mass% and more preferably 0 to 60 mass%.
If the content of the good solvent for the porous molded body-forming polymer is 80 mass% or lower, a favorable effect for a satisfactory porous molded body shape can be obtained.
The temperature of the poor solvent is preferably 40 to 100°C, more preferably 50 to 100°C and even more preferably 60 to 100°C, from the viewpoint of controlling the temperature and humidity of the spaces in step (4).

[Production apparatus for porous molded body]

When the porous molded body is in particulate form, the production apparatus comprises a rotating vessel that ejects droplets by centrifugal force and a coagulation tank that stores a coagulating solution, also optionally being provided with a cover that covers the space between the rotating vessel and the coagulation tank and comprising control means that controls the temperature and humidity in the space.
The rotating vessel that ejects droplets by centrifugal force is not restricted to one with a specific construction so long as it has the function of ejecting the molding slurry as spherical droplets by centrifugal force, and examples include known types of rotating discs or rotating nozzles.
With a rotating disc, the molding slurry is supplied to the center of the rotating disc and the molding slurry is developed into a film of uniform thickness along the surface of the rotating disc, and then divided into droplets by centrifugal force from the peripheral edges of the disc to eject the microdroplets.
A rotating nozzle either has a plurality of through-holes formed in the perimeter wall of a rotating vessel having a hollow disc shape, or it has nozzles attached through the perimeter wall, with the molding slurry being supplied into the rotating vessel while rotating the rotating vessel, and the molding slurry being discharged by centrifugal force from the through-holes or nozzles to form droplets.
The coagulation tank that stores the coagulating solution is not limited to any particular structure so long as it has a function allowing it to store the coagulating solution, and for example, it may be a coagulation tank with an open top side, as is commonly known, or a coagulation tank having a construction in which the coagulating solution is allowed to flow down naturally by gravity along the inner walls of the cylinder situated surrounding the rotating vessel.
A coagulation tank with an open top side is an apparatus that allows droplets ejected in the horizontal direction from the rotating vessel to fall down naturally, and traps droplets on the liquid surface of the coagulating solution stored in the open-top coagulation tank.
A coagulation tank with a construction in which the coagulating solution is allowed to flow down naturally by gravity along the inner walls of the cylinder surrounding the rotating vessel is an apparatus that discharges the coagulating solution at a roughly equivalent flow rate in the circumferential direction along the inner walls of the cylinder, and traps droplets in the coagulating solution flowing downward along the inner walls, causing them to coagulate.
The control means for the temperature and humidity in the space is provided with a cover that covers the space between the rotating vessel and coagulation tank, and it controls the temperature and humidity in the space.
The cover covering the space is not restricted to any particular construction so long as it has the function of isolating the space from the external environment and facilitating practical control of the temperature and humidity in the space, and it may be box-shaped, tubular or umbrella-shaped, for example.
The material of the cover may be stainless steel metal or plastic, for example. For isolation from the external environment, it may also be covered by a known type of insulation. The cover may also be partially provided with openings for temperature and humidity adjustment.
The means for controlling the temperature and humidity in the space is not limited to any particular means so long as it has the function of controlling the temperature and humidity in the space, and for example, it may be a heating machine such as an electric heater or steam heater, or a humidifier such as an ultrasonic humidifier or heating humidifier.

A preferred means in terms of construction is one that heats the coagulating solution stored in the coagulation tank and utilizes steam generated from the coagulating solution to control the temperature and humidity in the space.

A method of forming a coating layer of a biocompatible polymer on the surface of a porous molded body will now be described.

A coating solution containing a PMEA- or a PVP-based polymer, for example, may be applied onto the surface of the porous molded body to form a coating film. A PMEA coating solution, for example, can penetrate the pores formed in the porous molded body, allowing the PMEA to be added to the entire pore surface of the porous molded body without significantly altering the pore sizes on the surface of the porous molded article.

The solvent of the PMEA coating solution is not particularly restricted so long as it is one that can dissolve or disperse the PMEA without dissolving the polymers such as the porous molded body-forming polymer of the porous molded body and the water-soluble polymer, but it is preferably water or an aqueous alcohol solution, for process safety and satisfactory handleability in the subsequent drying step. From the viewpoint of the boiling point and of toxicity, it is preferred to use water, an aqueous ethanol solution, an aqueous methanol solution or an aqueous isopropyl alcohol solution.

The solvent of the PVP coating solution is not particularly restricted so long as it is a solvent that can dissolve or disperse the PVP without dissolving the polymers such as the porous molded body-forming polymer of the porous molded body and the water-soluble polymer, but it is preferably water or an aqueous alcohol solution, for process safety and satisfactory handleability in the subsequent drying step. From the viewpoint of the boiling point and of toxicity, it is preferred to use water, an aqueous ethanol solution, an aqueous methanol solution or an aqueous isopropyl alcohol solution.

The type and composition of the solvent in the coating solution is selected as appropriate in relation to the polymer forming the porous molded body.

The concentration of the PMEA coating solution is not restricted, but as an example it may be 0.001 mass% to 1 mass%, and preferably 0.005 mass% to 0.2 mass%, of the coating solution.

The method of applying the coating solution is also not restricted, and an example is a method in which the porous molded body is packed into a suitable column (vessel) and flushed from the top with a coating solution containing PMEA, and compressed air is then used to remove the excess solution.

After subsequently washing with distilled water and substituting out the unnecessary solvent, it may be sterilized for use as a medical tool.

EXAMPLES

The present invention will now be explained in greater detail by Examples, with the implicit understanding that the scope of the invention is not limited by the Examples, but defined by the appended claims.

The methods for measuring the physical property values mentioned above will now be explained.

[Mean particle size of porous molded body and mean particle size of inorganic ion adsorbent]

The mean particle size of the porous molded body and the mean particle size of the inorganic ion adsorbent are measured using a laser diffraction/scattering particle size distribution analyzer (LA-950, trade name of Horiba Co.). The dispersing medium used is water. For measurement of samples using hydrated cerium oxide as the inorganic ion adsorbent, the refractive index used is the value for cerium oxide. Likewise, for measurement of samples using hydrated zirconium oxide as the inorganic ion adsorbent, the refractive index used is the value for zirconium oxide.

[Phosphorus adsorption with bovine plasma]

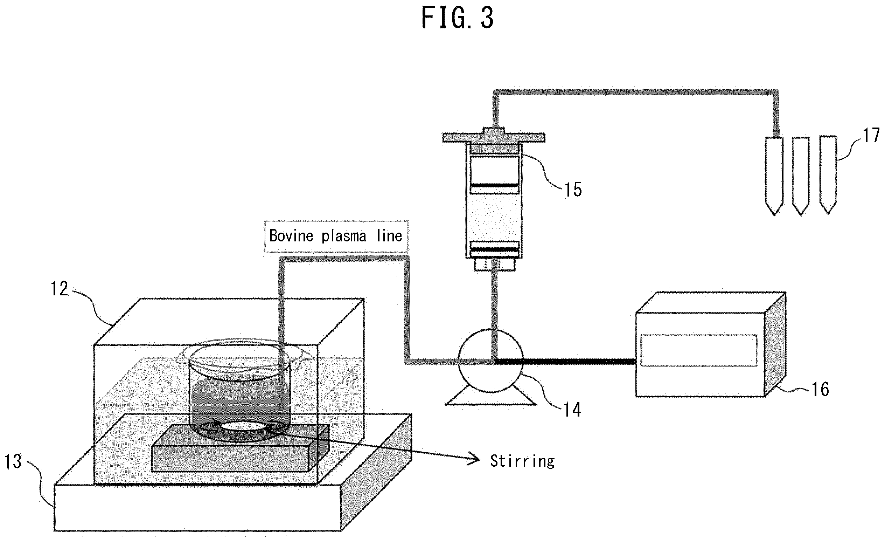

The apparatus shown in Fig. 3 is used to measure the phosphorus adsorption by a column flow test with low-phosphorus serum using bovine plasma. Bovine plasma with the phosphorus level adjusted to a low level (0.7 mg/dL) and stirred in a thermostatic bath (12) on a laboratory bench (13) is passed through a column (15) packed with a porous molded body using a pump (14) equipped with a pressure gauge (16), under conditions equivalent to common dialysis conditions (space velocity SV = 120, 4 hour dialysis), and upon sampling (17), the phosphorus adsorption (mg-P/mL-Resin (porous molded body)) of the porous molded body is measured.

The phosphate ion concentration is measured by the molybdic acid direct method.

Phosphorus adsorption of 1.5 (mg-P/mL-resin) or greater with a flow speed of SV120 is judged to be high adsorption capacity and satisfactory as a phosphorus adsorbent.

[Example 1]

A first embodiment of the invention will now be explained in detail with reference to Fig. 1. (A) and (B) are blood vessels of the patient. The blood circuit (1) includes the blood collection unit (1a) which is inserted into the blood vessel (A) of the patient and collects blood while the blood circuit (10) includes the blood returning unit (1b) which returns blood into the blood vessel (B) of the patient, the blood circuits (tubing systems) being made of vinyl tubes. The pump (2) is situated in the blood circuit (1). The pump (2) causes blood to be supplied to the blood component adjuster (4), or to the blood purification device (3) through the bypass tubing system (6). The blood purification device (3) has a dialysate inlet (3a) and a dialysate outlet (3b). The blood component adjuster (4) is situated further toward the blood collection unit (1a) side than the blood purification device (3). At the ends of the blood purification device (3) and blood component adjuster (4) there are provided pressure sensors (5 to 5′′′) which measure the blood pressure at the inlet and the filtrate pressure at the outlet. A bypass tubing system (6) is provided at both ends of the blood component adjuster (4). A valve (7) is provided in the bypass tubing system (6).

When the inlet pressure of the blood component adjuster (4) has increased above a predetermined value due to clogging or the like, or when the pressure loss has exceeded a predetermined value, the valve (7) is switched from closed to open based on data from the pressure sensors (5, 5′) at both ends of the blood component adjuster (4), thereby allowing blood to flow into the bypass tubing system (6) to prevent damage to the blood component adjuster (4) and blood circuit, and allowing safe operation. Two pressure sensors (5′, 5") are present between the blood purification device (3) and blood component adjuster (4) here, but a single one may be used instead.

When the pressure of the blood purification device (3) has increased above a predetermined level due to clogging or the like, or when the pressure loss has exceeded a predetermined value, the mode is switched from dialysis mode to reinfusion mode or a different mode by a command from the control unit, based on data from the pressure sensors (5", 5′′′) at both ends of the blood purification device (3). This can prevent damage to the blood purification device (3) and blood circuit, thus allowing safe operation to be carried out.

[Example 2]

Another embodiment of the invention will now be explained in detail with reference to Fig. 2.

(A) and (B) are blood vessels of the patient. The blood circuit (1) includes the blood collection unit (1a) which is inserted into the blood vessel (A) of the patient and collects blood while the blood circuit (10) includes the blood returning unit (1b) which returns blood into the blood vessel (B) of the patient, the blood circuits (tubing systems) being made of vinyl tubes. The pump (2) is situated in the blood circuit (1). The pump (2) causes blood to be supplied to the blood purification device (3), or to the blood component adjuster (4) through the bypass tubing system (6). The blood purification device (3) has a dialysate inlet (3a) and a dialysate outlet (3b). The blood component adjuster (4) is situated further toward the blood returning unit (1b) side than the blood purification device (3). At the ends of the blood purification device (3) and blood component adjuster (4) there are provided pressure sensors (5 to 5′′′) which measure the blood pressure at the inlet and the filtrate pressure at the outlet. A bypass tubing system (6) is provided at both ends of the blood purification device (3). A valve (7) is provided in the bypass tubing system (6).

When the inlet pressure of the blood purification device (3) has increased above a predetermined value due to clogging or the like, or when the pressure loss has exceeded a predetermined value, the valve (7) is switched from closed to open based on data from the pressure sensors (5", 5′′′) at both ends of the blood purification device (3), thereby allowing blood to flow into the bypass tubing system (6) to prevent damage to the blood purification device (3) and blood circuit, and allowing safe operation. Two pressure sensors (5, 5′′′) are present between the blood purification device (3) and blood component adjuster (4) here, but a single one may be used instead.

When the pressure of the blood component adjuster (4) has increased above a predetermined level due to clogging or the like, or when the pressure loss has exceeded a predetermined value, the mode is switched from dialysis mode to reinfusion mode or a different mode by a command from the control unit, based on data from the pressure sensors (5, 5′) at both ends of the blood component adjuster (4). This can prevent damage to the blood component adjuster (4) and blood circuit, thus allowing safe operation to be carried out.

INDUSTRIAL APPLICABILITY

The extracorporeal blood circulation system of the invention can be safely used since it switches dialysis mode to reinfusion mode and bypasses the blood circuit based on pressure loss of the blood purification device and blood component adjuster, thereby making it possible to avoid damage to the blood purification device, blood component adjuster and blood circuit (tubing system).

REFERENCE SIGNS LIST

A Patient blood vessel
B Patient blood vessel
C Reservoir
C' Reservoir
1 Tubing system (blood circuit)
1a Blood collection unit
1b Blood returning unit
2 Pump
3 Blood purification device
3a Dialysate inlet
3b Dialysate outlet
4 Blood component adjuster
5 Pressure gauge (sensor)
5' Pressure gauge
5" Pressure gauge
5‴ Pressure gauge
6 Bypass tubing system (blood circuit)
7 Valve
8 (Three-way) valve
8' (Three-way) valve
9 Tubing system (blood circuit)
10 Tubing system (blood circuit)
11 Tubing system
11' Tubing system
12 Thermostatic bath
13 Laboratory bench
14 Pump
15 Column containing phosphorus absorbent
16 Pressure gauge
17 Sampling

**Claims**

1. An extracorporeal blood circulation system running from a blood collection unit (1a) to a blood returning unit (1b), wherein the extracorporeal blood circulation system comprises the following:

   a blood component adjuster (4);
   a blood purification device (3);
   a tubing system (1) comprising a pump (2) for supply of blood from the blood collection unit (1a) to the blood component adjuster (4) in dialysis mode, a valve (8) for supply of physiological saline or air from a tubing system (11) in place of blood, in reinfusion mode, and a pressure gauge (5) for detection of pressure loss of the blood component adjuster (4);
   a bypass tubing system (6) comprising a valve (7) for supply of blood to the blood purification device (3) bypassing the blood component adjuster (4), and supply of physiological saline or air in reinfusion mode;
   a tubing system (9) which comprises pressure gauges (5', 5") for detecting pressure loss of the blood component adjuster (4) and/or blood purification device (3), and connects the blood component adjuster (4) and the blood purification device (3);
   a tubing system (10) comprising a pressure gauge (5‴) for returning blood from the blood purification device (3) to the blood returning unit (1b) and for detecting pressure loss of the blood purification device (3), in dialysis mode, and if necessary a valve (8') for recovering physiological saline or air in the tubing system (11') in place of blood, in reinfusion mode; and
   a control unit having a function for switching between the tubing system (1) and the bypass tubing system (6), based on pressure loss of the blood component adjuster (4), and a function for switching between dialysis mode and reinfusion mode, based on pressure loss of the blood purification device (3).

2. An extracorporeal blood circulation system running from a blood collection unit (1a) to a blood returning unit (1b), wherein the extracorporeal blood circulation system comprises the following:

a blood purification device (3);
a blood component adjuster (4);
a tubing system (1) comprising a pump (2) for supply of blood from the blood collection unit (1a) to the blood purification device (3) in dialysis mode, a valve (8) for supply of physiological saline or air from a tubing system (11) in place of blood, in reinfusion mode, and a pressure gauge (5") for detection of pressure loss of the blood purification device (3);
a bypass tubing system (6) comprising a valve (7) for supply of blood to the blood component adjuster (4) bypassing the blood purification device (3), and supply of physiological saline or air in reinfusion mode;
a tubing system (9) which comprises pressure gauges (5, 5‴) for detecting pressure loss of the blood purification device (3) and/or blood component adjuster (4), and which connects the blood purification device (3) and the blood component adjuster (4);
a tubing system (10) comprising a pressure gauge (5') for returning blood from the blood component adjuster (4) to the blood returning unit (1b) and for detecting pressure loss of the blood component adjuster (4), in dialysis mode, and if necessary a valve (8') for recovering physiological saline or air in the tubing system (11') in place of blood, in reinfusion mode; and
a control unit having a function for switching between the tubing system (1) and the bypass tubing system (6), based on pressure loss of the blood purification device (3), and a function for switching between dialysis mode and reinfusion mode, based on pressure loss of the blood component adjuster (4).

3. The extracorporeal blood circulation system according to claim 1 or 2, wherein the blood component adjuster (4) has a blood component adjusting body.

4. The extracorporeal blood circulation system according to claim 3, wherein the blood component adjusting body is a porous molded body.

5. The extracorporeal blood circulation system according to claim 4, wherein the porous molded body is composed of a porous molded body-forming polymer and a hydrophilic polymer, or is composed of a porous molded body-forming polymer, a hydrophilic polymer and an inorganic ion adsorbent.

6. The extracorporeal blood circulation system according to claim 5, wherein the porous molded body-forming polymer is an aromatic polysulfone.

7. The extracorporeal blood circulation system according to claim 5 or 6, wherein the hydrophilic polymer is a biocompatible polymer.

8. The extracorporeal blood circulation system according to claim 7, wherein the biocompatible polymer is a polyvinylpyrrolidone (PVP)-based polymer.

9. The extracorporeal blood circulation system according to any one of claims 4 to 8, wherein the porous molded body is coated with a biocompatible polymer.

10. The extracorporeal blood circulation system according to claim 9, wherein the biocompatible polymer is selected from the group consisting of polyvinylpyrrolidone (PVP)-based polymers and polymethoxyethyl acrylate (PMEA).

11. The extracorporeal blood circulation system according to any one of claims 4 to 10, wherein the blood phosphorus adsorption of the porous molded body is 2 (mg-P/mL-Resin) or greater.

12. The extracorporeal blood circulation system according to any one of claims 5 to 11, wherein the inorganic ion adsorbent contains at least one metal oxide represented by the following formula (I):

$$MN_xO_n \cdot mH_2O \qquad (I)$$

{where x is 0 to 3, n is 1 to 4, m is 0 to 6, and M and N are metal elements selected from the group consisting of Ti, Zr, Sn, Sc, Y, La, Ce, Pr, Nd, Sm, Eu, Gd, Tb, Dy, Ho, Er, Tm, Yb, Lu, Al, Si, Cr, Co, Ga, Fe, Mn, Ni, V, Ge, Nb and Ta, and are different from each other}.

13. The extracorporeal blood circulation system according to claim 12, wherein the metal oxide is selected from among the following groups (a) to (c):

(a) hydrated titanium oxide, hydrated zirconium oxide, hydrated tin oxide, hydrated cerium oxide, hydrated lanthanum oxide and hydrated yttrium oxide;
(b) complex metal oxides comprising at least one metal element selected from the group consisting of titanium, zirconium, tin, cerium, lanthanum and yttrium and at least one metal element selected from the group consisting of aluminum, silicon and iron; and
(c) activated alumina.

**Patentansprüche**

1. Extrakorporales Blutkreislaufsystem, das von einer Blutsammeleinheit (1a) zu einer Blutrückführungseinheit (1b) verläuft, wobei das extrakorporale Blutkreislaufsystem Folgendes umfasst:

eine Blutkomponenten-Einstellvorrichtung (4);
eine Blutreinigungsvorrichtung (3);
ein Rohrleitungssystem (1), umfassend eine Pumpe (2) zum Zuführen von Blut aus der Blutsammeleinheit (1a) zu der Blutkomponenten-Einstellvorrichtung (4) im Dialysemodus, ein Ventil (8) zum Zuführen von physiologischer Kochsalzlösung oder Luft aus einem Rohrleitungssystem (11) anstelle von Blut im Reinfusionsmodus und ein Manometer (5) zum Nachweis eines Druckverlusts bei der Blutkomponenten-Einstellvorrichtung (4);
ein Rohrumleitungssystem (6), umfassend ein Ventil (7) zum Zuführen von Blut zu der Blutreinigungsvorrichtung (3) unter Umgehung der Blutkomponenten-Einstellvorrichtung (4) und Zuführen von physiologischer Kochsalzlösung oder Luft im Reinfusionsmodus;
ein Rohrleitungssystem (9), das Manometer (5', 5") umfasst, um einen Druckverlust der Blutkomponenten-Einstellvorrichtung (4) und/oder der Blutreinigungsvorrichtung (3) nachzuweisen, und die Blutkomponenten-Einstellvorrichtung (4) und die Blutreinigungsvorrichtung (3) miteinander verbindet;
ein Rohrleitungssystem (10), umfassend ein Manometer (5‴) zum Rückführen von Blut aus der Blutreinigungsvorrichtung (3) zu der Blutrückführungseinheit (1b) und zum Nachweis eines Druckverlusts der Blutreinigungsvorrichtung (3) im Dialysemodus und gegebenenfalls ein Ventil (8') zum Rückgewinnen von physiologischer Kochsalzlösung oder Luft in dem Rohrleitungssystem (11') anstelle von Blut im Reinfusionsmodus und eine Steuerungseinheit mit der Funktion, zwischen dem Rohrleitungssystem (1) und dem Rohrumleitungssystem (6) hin und her zu schalten, und zwar auf der Basis des Druckverlusts der Blutkomponenten-Einstellvorrichtung (4), und der Funktion, zwischen dem Dialysemodus und dem Reinfusionsmodus hin und her zu schalten, und zwar auf der Basis des Druckverlusts der Blutreinigungsvorrichtung (3).

2. Extrakorporales Blutkreislaufsystem, das von einer Blutsammeleinheit (1a) zu einer Blutrückführungseinheit (1b) verläuft, wobei das extrakorporale Blutkreislaufsystem Folgendes umfasst:

eine Blutreinigungsvorrichtung (3);
eine Blutkomponenten-Einstellvorrichtung (4);
ein Rohrleitungssystem (1), umfassend eine Pumpe (2) zum Zuführen von Blut aus der Blutsammeleinheit (1a) zu der Blutreinigungsvorrichtung (3) im Dialysemodus, ein Ventil (8) zum Zuführen von physiologischer Kochsalzlösung oder Luft aus einem Rohrleitungssystem (11) anstelle von Blut im Reinfusionsmodus und ein Manometer (5") zum Nachweis eines Druckverlusts bei der Blutreinigungsvorrichtung (3);
ein Rohrumleitungssystem (6), umfassend ein Ventil (7) zum Zuführen von Blut zu der Blutkomponenten-Einstellvorrichtung (4) unter Umgehung der Blutreinigungsvorrichtung (3) und Zuführen von physiologischer Kochsalzlösung oder Luft im Reinfusionsmodus;
ein Rohrleitungssystem (9), das Manometer (5, 5‴) umfasst, um einen Druckverlust der Blutreinigungsvorrichtung (3) und/oder der Blutkomponenten-Einstellvorrichtung (4) nachzuweisen, und die Blutreinigungsvorrichtung (3) und die Blutkomponenten-Einstellvorrichtung (4) miteinander verbindet;
ein Rohrleitungssystem (10), umfassend ein Manometer (5') zum Rückführen von Blut aus der Blutkomponenten-Einstellvorrichtung (4) zu der Blutrückführungseinheit (1b) und zum Nachweis eines Druckverlusts der Blutkomponenten-Einstellvorrichtung (4) im Dialysemodus und gegebenenfalls ein Ventil (8') zum Rückgewinnen von physiologischer Kochsalzlösung oder Luft in dem Rohrleitungssystem (11') anstelle von Blut im Reinfusionsmodus und

eine Steuerungseinheit mit der Funktion, zwischen dem Rohrleitungssystem (1) und dem Rohrumleitungssystem (6) hin und her zu schalten, und zwar auf der Basis des Druckverlusts der Blutreinigungsvorrichtung (3), und der Funktion, zwischen dem Dialysemodus und dem Reinfusionsmodus hin und her zu schalten, und zwar auf der Basis des Druckverlusts der Blutkomponenten-Einstellvorrichtung (4).

3. Extrakorporales Blutkreislaufsystem gemäß Anspruch 1 oder 2, wobei die Blutkomponenten-Einsteilvorrichtung (4) einen Blutkomponenten-Einstellkörper aufweist.

4. Extrakorporales Blutkreislaufsystem gemäß Anspruch 3, wobei der Blutkomponenten-Einstellkörper ein poröser Formkörper ist.

5. Extrakorporales Blutkreislaufsystem gemäß Anspruch 4, wobei der poröse Formkörper aus einem den porösen Formkörper bildenden Polymer und einem hydrophilen Polymer besteht oder aus einem den porösen Formkörper bildenden Polymer, einem hydrophilen Polymer und einem anorganischen Ionen-Adsorber besteht.

6. Extrakorporales Blutkreislaufsystem gemäß Anspruch 5, wobei das den porösen Formkörper bildende Polymer ein aromatisches Polysulfon ist.

7. Extrakorporales Blutkreislaufsystem gemäß Anspruch 5 oder 6, wobei das hydrophile Polymer ein bioverträgliches Polymer ist.

8. Extrakorporales Blutkreislaufsystem gemäß Anspruch 7, wobei das bioverträgliche Polymer ein Polymer auf Polyvinylpyrrolidon(PVP)-Basis ist.

9. Extrakorporales Blutkreislaufsystem gemäß einem der Ansprüche 4 bis 8, wobei der poröse Formkörper mit einem bioverträglichen Polymer beschichtet ist.

10. Extrakorporales Blutkreislaufsystem gemäß Anspruch 9, wobei das bioverträgliche Polymer aus der Gruppe ausgewählt ist, die aus Polymeren auf Polyvinylpyrrolidon(PVP)-Basis und Polymethoxyethylacrylat (PMEA) besteht.

11. Extrakorporales Blutkreislaufsystem gemäß einem der Ansprüche 4 bis 10, wobei die Blutphosphoradsorption des porösen Formkörpers 2 (mg P/ml Harz) oder mehr beträgt.

12. Extrakorporales Blutkreislaufsystem gemäß einem der Ansprüche 5 bis 11, wobei der anorganische Ionenadsorber wenigstens ein Metalloxid enthält, das durch die folgende Formel (I) dargestellt wird:

$$MN_xO_n \cdot mH_2O \qquad (I)$$

wobei x = 0 bis 3 ist, n = 1 bis 4 ist, m = 0 bis 6 ist und M und N Metallelemente sind, die aus der Gruppe ausgewählt sind, die aus Ti, Zr, Sn, Sc, Y, La, Ce, Pr, Nd, Sm, Eu, Gd, Tb, Dy, Ho, Er, Tm, Yb, Lu, Al, Si, Cr, Co, Ga, Fe, Mn, Ni, V, Ge, Nb und Ta besteht, und voneinander verschieden sind.

13. Extrakorporales Blutkreislaufsystem gemäß Anspruch 12, wobei das Metalloxid aus den folgenden Gruppen (a) bis (c) ausgewählt ist:

(a) Titanoxidhydrat, Zirconiumoxidhydrat, Zinnoxidhydrat, Ceroxidhydrat, Lanthanoxidhydrat und Yttriumoxidhydrat besteht;
(b) komplexen Metalloxiden, die wenigstens ein Metallelement umfassen, das aus der Gruppe ausgewählt ist, die aus Titan, Zirconium, Zinn, Cer, Lanthan und Yttrium und wenigstens einem Metallelement, das aus der Gruppe ausgewählt ist, Aluminium, Silicium und Eisen besteht; und
(c) aktivierter Tonerde.

**Revendications**

1. Système de circulation de sang extracorporel allant d'une unité de recueil de sang (1a) jusqu'à une unité de renvoi de sang (1b), dans lequel le système de circulation de sang extracorporel comprend les suivants :

un dispositif de réglage de composant sanguin (4) ;

un dispositif de purification de sang (3) ;

un système de tuyauterie (1) comprenant une pompe (2) pour introduire du sang à partir de l'unité de recueil de sang (1a) dans le dispositif de réglage de composant sanguin (4) en mode dialyse, une vanne (8) pour introduire une solution saline physiologique ou de l'air à partir d'un système de tuyauterie (11) à la place du sang, en mode réinfusion, et un manomètre (5) pour une détection de la perte de pression du dispositif de réglage de composant sanguin (4) ;

un système de tuyauterie de dérivation (6) comprenant une vanne (7) pour introduire du sang dans le dispositif de purification de sang (3) court-circuitant le dispositif de réglage de composant sanguin (4), et introduire une solution saline physiologique ou de l'air en mode réinfusion ;

un système de tuyauterie (9) qui comprend des manomètres (5', 5") pour détecter une perte de pression du dispositif de réglage de composant sanguin (4) et/ou dispositif de purification de sang (3), et raccorde le dispositif de réglage de composant sanguin (4) et le dispositif de purification de sang (3) ;

un système de tuyauterie (10) comprenant un manomètre (5‴) pour renvoyer du sang du dispositif de purification de sang (3) dans l'unité de renvoi de sang (1b) et pour détecter une perte de pression du dispositif de purification de sang (3), en mode dialyse, et si nécessaire une vanne (8') pour récupérer une solution saline physiologique ou de l'air dans le système de tuyauterie (11') à la place du sang, en mode réinfusion ; et

une unité de contrôle ayant une fonction de commutation entre le système de tuyauterie (1) et le système de tuyauterie de dérivation (6), sur la base d'une perte de pression du dispositif de réglage de composant sanguin (4), et une fonction de commutation entre un mode dialyse et un mode réinfusion, sur la base de la perte de pression du dispositif de purification de sang (3).

2. Système de circulation de sang extracorporel allant d'une unité de recueil de sang (1a) jusqu'à une unité de renvoi de sang (1b), dans lequel le système de circulation de sang extracorporel comprend les suivants :

un dispositif de purification de sang (3) ;

un dispositif de réglage de composant sanguin (4) ;

un système de tuyauterie (1) comprenant une pompe (2) pour introduire du sang de l'unité de recueil de sang (1a) dans le dispositif de purification de sang (3) en mode dialyse, une vanne (8) pour introduire une solution saline physiologique ou de l'air à partir d'un système de tuyauterie (11) à la place du sang, en mode réinfusion, et un manomètre (5") pour une détection de perte de pression du dispositif de purification de sang (3) ;

un système de tuyauterie de dérivation (6) comprenant une vanne (7) pour introduire du sang dans le dispositif de réglage de composant sanguin (4) court-circuitant le dispositif de purification de sang (3), et introduire une solution saline physiologique ou de l'air en mode réinfusion ;

un système de tuyauterie (9) qui comprend des manomètres (5, 5‴) pour détecter une perte de pression du dispositif de purification de sang (3) et/ou dispositif de réglage de composant sanguin (4), et qui raccorde le dispositif de purification de sang (3) et le dispositif de réglage de composant sanguin (4) ;

un système de tuyauterie (10) comprenant un manomètre (5') pour renvoyer du sang du dispositif de réglage de composant sanguin (4) dans l'unité de renvoi de sang (1b) et pour détecter une perte de pression du dispositif de réglage de composant sanguin (4), en mode dialyse, et si nécessaire une vanne (8') pour récupérer une solution saline physiologique ou de l'air dans le système de tuyauterie (11') à la place du sang, en mode réinfusion ; et

une unité de contrôle ayant une fonction de commutation entre le système de tuyauterie (1) et le système de tuyauterie de dérivation (6), sur la base d'une perte de pression du dispositif de purification de sang (3), et une fonction de commutation entre un mode dialyse et un mode réinfusion, sur la base d'une perte de pression du dispositif de réglage de composant sanguin (4).

3. Système de circulation de sang extracorporel selon la revendication 1 ou 2, dans lequel le dispositif de réglage de composant sanguin (4) présente un corps de réglage de composant sanguin.

4. Système de circulation de sang extracorporel selon la revendication 3, dans lequel le corps de réglage de composant sanguin est un corps moulé poreux.

5. Système de circulation de sang extracorporel selon la revendication 4, dans lequel le corps moulé poreux est constitué d'un polymère formant un corps moulé poreux et d'un polymère hydrophile, ou est constitué d'un polymère formant un corps moulé poreux, d'un polymère hydrophile et d'un adsorbant d'ions inorganiques.

**6.** Système de circulation de sang extracorporel selon la revendication 5, dans lequel le polymère formant un corps moulé poreux est une polysulfone aromatique.

**7.** Système de circulation de sang extracorporel selon la revendication 5 ou 6, dans lequel le polymère hydrophile est un polymère biocompatible.

**8.** Système de circulation de sang extracorporel selon la revendication 7, dans lequel le polymère biocompatible est un polymère à base de polyvinylpyrrolidone (PVP).

**9.** Système de circulation de sang extracorporel selon l'une quelconque des revendications 4 à 8, dans lequel le corps moulé poreux est revêtu d'un polymère biocompatible.

**10.** Système de circulation de sang extracorporel selon la revendication 9, dans lequel le polymère biocompatible est choisi dans le groupe consistant en polymères à base de polyvinylpyrrolidone (PVP) et poly(acrylate de méthoxyéthyle) (PMEA).

**11.** Système de circulation de sang extracorporel selon l'une quelconque des revendications 4 à 10, dans lequel l'adsorption de phosphore du sang du corps moulé poreux est de 2 (mg-P/mL-résine) ou supérieure.

**12.** Système de circulation de sang extracorporel selon l'une quelconque des revendications 5 à 11, dans lequel l'adsorbant d'ions inorganiques contient au moins un oxyde de métal représenté par la formule (I) suivante :

$$MN_xO_n.mH_2O \qquad (I)$$

{où x est de 0 à 3, n est de 1 à 4, m est de 0 à 6, et M et N sont des éléments de métaux choisis dans le groupe consistant en Ti, Zr, Sn, Sc, Y, La, Ce, Pr, Nd, Sm, Eu, Gd, Tb, Dy, Ho, Er, Tm, Yb, Lu, Al, Si, Cr, Co, Ga, Fe, Mn, Ni, V, Ge, Nb et Ta, et sont différents l'un de l'autre}.

**13.** Système de circulation de sang extracorporel selon la revendication 12, dans lequel l'oxyde de métal est choisi parmi les groupes (a) à (c) suivants :

(a) oxyde de titane hydraté, oxyde de zirconium hydraté, oxyde d'étain hydraté, oxyde de cérium hydraté, oxyde de lanthane hydraté et oxyde d'yttrium hydraté ;
(b) oxydes de métaux complexes comprenant au moins un élément de métal choisi dans le groupe consistant en titane, zirconium, étain, cérium, lanthane et yttrium et au moins un élément de métal choisi dans le groupe consistant en aluminium, silicium et fer ; et
(c) alumine activée.

# FIG. 1

# FIG. 2

FIG. 3

Bovine plasma line

Stirring

EP 3 903 855 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2018326136 A1
- DE 102017216689 A1
- WO 2018180043 A1
- US 2016339161 A1
- CN 105107041 A

### Non-patent literature cited in the description

- Artificial organ surface-biocompatibilizing materials. **TANAKA, K.** BIO INDUSTRY. 2003, vol. 20, 59-70